# NOUVEAU FASCICULE DE BREVET EUROPEEN

(11) **EP 1 522 319 B2**
(45) Date de publication et mention de la décision concernant l'opposition: **13.10.2010**
(45) Mention de la délivrance du brevet: 09.01.2008
(21) Numéro de dépôt: 04077788.0
(22) Date de dépôt: 08.10.2004
(51) Int. Cl.: A61L 9/05, E03D 9/00, C11D 17/04, A61L 9/12

(54) **Dispositif hygiénique avec élément parfumant pour sanitaires**
Hygienevorrichtung mit Duftelement für Sanitäranlagen
Hygienic device with perfuming element for sanitary arrangements

(30) Priorité: 10.10.2003 IT MI20030472; 08.01.2004 FR 0400135
(43) Date de publication de la demande: 13.04.2005
(73) Titulaire: Bolton Manitoba SpA, 20124 Milano (IT)
(72) Inventeur: Luciani, Alain, 20054 Nova Milanese (Milano) (IT); Ferri Michele, 20059 Vimercate (Milano) (IT); Pasquarella, Sandra, 20010 Vittuone (Milano) (IT); Oliva, Marco, 20155 Milano (IT)
(74) Mandataire: De Gregori, Antonella

(56) Documents cités:
- EP-A- 0 014 979
- WO- -03/027405
- WO- -03/042462
- DE-A- 2 833 770
- DE-A- 19 830 308
- US-A- 3 504 384
- US-A- 3 715 765
- US-A- 4 670 916

## Description

La présente invention concerne un dispositif hygiénique avec un élément parfumant pour les sanitaires.

Dans les appareils sanitaires, comme une cuvette de WC, le nettoyage et la désinfection se dégradent rapidement provoquant, de ce fait, non seulement des conditions d'utilisation déplaisantes et antihygiéniques, mais aussi une dégradation desdits sanitaires due à la formation de calcaire et d'autres incrustations.

Les dispositifs hygiéniques qui s'installent à l'intérieur des toilettes sont très courants. Ils sont retenus à l'aide d'un système de suspension sur un bord des toilettes et ils sont atteints par l'eau de la chasse.

Ces dispositifs hygiéniques peuvent être du type "à cage", c'est-à-dire comprenant un élément contenant des fissures et/ou des trous qui permettent le passage de l'eau de la chasse, et dans lequel se trouve un ou des composés chimiques actifs à l'état solide, par exemple avec des fonctions détergentes et déodorantes.

En alternative, des dispositifs hygiéniques comprenant un flacon contenant un ou des composés actifs chimiques liquides ou en gel ont été aussi développés, appelés ici dispositifs "à flacon". Les flacons, qui peuvent être interchangeables, sont disposés sur des distributeurs qui sont capables de libérer une quantité contrôlée de composés actifs à chaque flux de la chasse d'eau.

L'eau de la chasse s'enrichit, grâce au dispositif hygiénique, de substances qui lui confèrent des propriétés par exemple détergentes, décalcifiantes, déodorantes et désinfectantes, ce qui augmente de manière importante son action nettoyante naturelle.

Cependant, les dispositifs hygiéniques connus pour sanitaires ne résolvent pas efficacement le problème de l'émanation dans l'environnement des mauvaises odeurs venant des toilettes.

En effet, l'effet parfumant et/ou déodorant des substances actives émises dans l'eau n'est pas suffisant pour masquer les odeurs et, surtout, il se maintient difficilement dans le temps jusqu'au flux suivant de la chasse d'eau. Il est donc souvent nécessaire d'ajouter dans la pièce un déodorant d'intérieur.

Le document US-4670916 décrit un dispositif hygiénique avec élément parfumant pour sanitaires, d'un type adapté pour être installé à l'intérieur et sur le bord des toilettes, comprenant, d'une part, un élément distributeur d'un composant chimique actif, qui relâche dans le flux de la chasse d'eau une quantité déterminée dudit composant actif, et, d'autre part, un récipient auxiliaire, le récipient auxiliaire comprenant un réservoir contenant un élément contenant une substance parfumante volatile.

Les solutions jusqu'à maintenant proposées pour parfumer les sanitaires à l'aide de dispositifs hygiéniques sont encore insuffisantes comparées à la demande.

Le but de la présente invention est de réaliser un dispositif hygiénique avec un élément parfumant pour appareils sanitaires qui contribue à résoudre les inconvénients mentionnés ci-dessus.

Un autre but de l'invention est de réaliser un dispositif hygiénique avec un élément parfumant pour appareils sanitaires facile à utiliser et qui puisse être accroché à tous types de sanitaires vendus dans le commerce.

La présente invention a donc pour objet un dispositif hygiénique selon la revendication 1.

Le réservoir peut être en tout matériau approprié connu de l'homme de l'art, notamment en plastique, par exemple en polyéthylène téréphtalate (PET).

L'élément distributeur est du type "à cage", et peut être en tout matériau approprié connu de l'homme de l'art.

Par composant chimique actif, on entend un composant ayant une action de type détergent, désinfectant, déodorant, parfumant et/ou autre.

Par substance parfumante, on entend une substance ayant une action parfumante et/ou désodorisante.

Par membrane semi-perméable, on entend une membrane qui est imperméable à l'élément contenant la ou les substances volatiles, et est perméable à la ou aux substances volatiles.

L'élément contenant une ou des substances parfumantes volatiles peut être de tout type approprié, en particulier solide, pâteux, caoutchouteux, liquide ou gel.

Le dispositif est de type adapté pour être installé sur des sanitaires, en ce sens qu'il comprend un moyen de fixation, de préférence amovible, sur le bord de la cuvette des sanitaires, de façon à être au moins partiellement atteint par le flux de la chasse d'eau.

Dans un mode de réalisation, la membrane semi-perméable comprend au moins une couche de polyéthylène perméable aux substances volatiles. Elle peut être co-extrudée avec la couche de protection.

Dans un mode de réalisation, la couche de protection hydrosoluble comprend une pellicule de polyvinylalcool (PVOH). Elle peut être fixée à la membrane semi-perméable par une couche de colle.

Le récipient auxiliaire est activé, c'est-à-dire prêt à libérer les substances parfumantes volatiles dans l'environnement, quand un flux d'eau atteint la couche de protection hydrosoluble provoquant sa dissolution.

Le récipient auxiliaire peut être appliqué de façon fixe ou amovible sur l'élément distributeur.

Dans un mode de réalisation, le récipient auxiliaire est appliqué sur l'élément distributeur par pression dans une base ou ouverture de forme complémentaire à celle du récipient auxiliaire.

L'élément distributeur comprend un récipient en matière plastique muni de multiples trous et/ou fissures pour le passage de l'eau et contenant ledit composé actif qui est solide ou pâteux, la base ou ouverture étant réalisée sur la partie antérieure dudit élément distributeur, le récipient auxiliaire comprenant la ou les substances parfumantes étant placé dans ladite base avec la membrane semi-perméable dirigée vers la partie interne de l'élément distributeur. Dans un mode de réalisation particulier, la partie antérieure de l'élément distributeur est pourvue d'au moins un picot dépassant de la surface interne et adapté pour maintenir ledit composé actif solide ou pâteux à distance du récipient auxiliaire et ainsi ne pas gêner la libération des substances parfumantes. De plus, le réservoir dudit récipient auxiliaire peut comprendre un périmètre dont une partie est munie d'au moins un trou en position complémentaire du au moins un picot pour un positionnement stable.

Par "partie antérieure", on entend la partie située du côté du bord de la cuvette des sanitaires, par opposition à la partie externe.

La couche de protection du récipient auxiliaire est généralement atteinte par le flux d'eau de la chasse d'eau quand le dispositif est appliqué sur le bord de la cuvette des sanitaires. Le flux de la chasse d'eau provoque donc l'élimination de la couche de protection hydrosoluble, mais n'est pas par la suite responsable de la libération des substances parfumantes, qui advient indépendamment du contact de la membrane semi-perméable avec l'eau.

Si la membrane semi-perméable du récipient auxiliaire est dans une position sur l'élément distributeur qui n'est pas atteinte par la chasse d'eau, alors l'exposition pendant un court moment du récipient auxiliaire à un flux d'eau avant son positionnement sur le bord de la cuvette des toilettes provoquera l'élimination de la couche de protection hydrosoluble et donc l'activation du récipient auxiliaire.

Après élimination de la couche de protection, l'évaporation des substances parfumantes dans l'atmosphère se fait sans interruption jusqu'à épuisement, et en particulier de manière indépendante du flux de la chasse d'eau. Par conséquent, si le composant chimique actif contenu dans l'élément distributeur devait être épuisé de façon rapide en raison de la fréquence élevée de flux de la chasse d'eau, le dispositif hygiénique selon l'invention pourrait être encore utilisé jusqu'à la libération totale des substances parfumantes. On peut également prévoir que l'élément distributeur contiennent aussi des substances parfumantes pour garantir l'effet parfumant désiré même si les substances parfumantes contenues dans le récipient auxiliaire sont épuisées avant le composant chimique actif.

Les caractéristiques et les avantages d'un dispositif hygiénique avec élément parfumant pour sanitaires selon l'invention seront discutés plus en détail dans la description suivante de modes de réalisation non limitatifs, relativement aux figures dans lesquelles :
La figure 1 est une vue d'un premier mode de réalisation d'un dispositif hygiénique avec élément parfumant pour sanitaires selon l'invention ;
La figure 2 est une vue du dispositif hygiénique de la figure 1 selon la coupe II-II ;
La figure 3 est une vue de coté du dispositif de la figure 1 ;
La figure 4 est une vue d'en haut du dispositif de la figure 1 partiellement coupée ;
Les figures 5 et 6 montrent un élément parfumant du dispositif hygiénique de la figure 1.

En faisant référence aux figures, il est représenté un dispositif hygiénique selon l'invention, désigné dans son ensemble par la référence 10 ou 10'. Ledit dispositif est adapté pour être installé à l'intérieur des toilettes ou WC, suspendu à un bord du périmètre supérieur, à titre d'exemple au moyen d'un élément accrocheur 12, comprenant un élément flexible en plastique pouvant s'appliquer de façon universelle à cheval sur le bord supérieur du périmètre des toilettes.

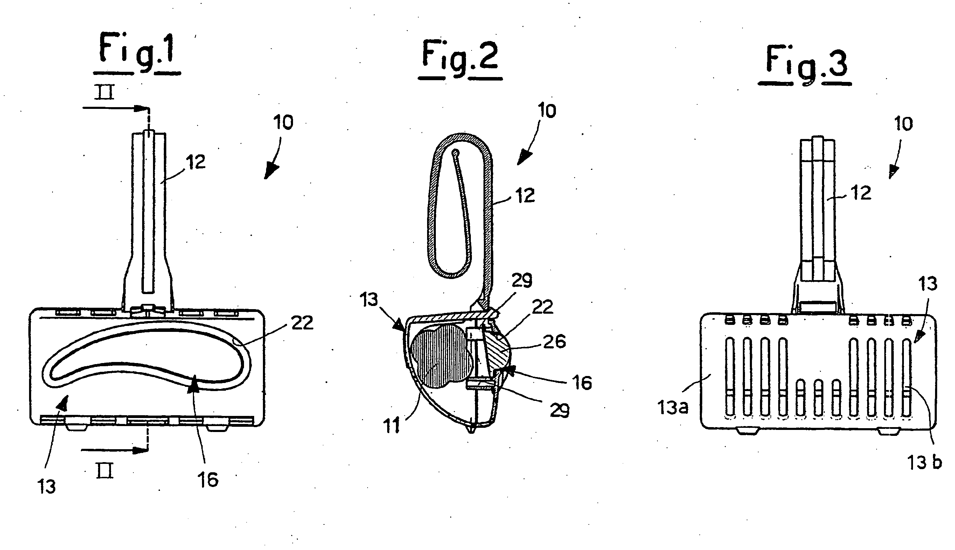

Dans le mode de réalisation représenté en figures 1-6, le dispositif hygiénique 10 comprend un élément distributeur 13 qui est capable de relâcher, dans le flux d'eau, une quantité déterminée d'au moins un composé actif chimique.

Le composé actif chimique peut être à l'état solide ou pâteux, l'élément distributeur 13 peut alors être un élément distributeur "à cage". Il peut notamment comprendre un récipient 13a, par exemple en plastique, pourvu de trous et/ou fissures 13b pour le passage de l'eau (cf. figure 3). En particulier, le composé actif chimique peut être sous forme de savonette 11 (cf. figure 2).

Le dispositif 10 comprend, de plus, des moyens pour la libération dans l'air de substances parfumantes volatiles. Lesdits moyens comprennent un récipient auxiliaire 16. Le récipient auxiliaire 16 comprend un réservoir 26, qui peut être une coquille en plastique, par exemple en polyéthylène térephtalate, contenant la ou les substances parfumantes volatiles, et sur lequel est appliquée une membrane semi-perméable 23. La membrane semi-perméable 23 est recouverte d'une couche de protection 18.

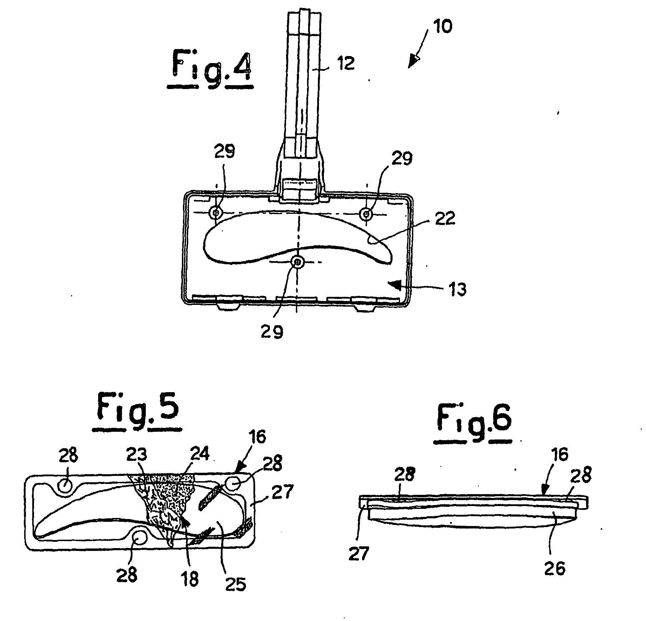

Ladite couche 18 est représentée schématiquement en figure 5 : la couche 18, qui est partiellement enlevée, comprend une couche de matériel hydrosoluble 25, par exemple une pellicule de polyvinylalcool, en contact avec la membrane semi-perméable 23 au moyen d'une couche de colle 24, qui est enlevée en même temps que la couche de protection est dissoute.

Un exemple de récipient auxiliaire 16 pour substances parfumantes volatiles comprend un périmètre 27 et un réservoir 26, la membrane semi-perméable 23 et la couche de protection 18 étant appliquées sur le périmètre 27.

Sur le périmètre 27 du récipient 16, trois trous 28 sont creusés pour le placement stable du récipient 16 sur la surface interne du côté antérieur de l'élément distributeur à cage 13, la surface interne étant munie de trois picots 29 en saillie en position complémentaire des trois trous. Une ouverture 22 de forme complémentaire au réservoir 26 du récipient auxiliaire 16 est réalisée dans la partie antérieure de l'élément distributeur 13, ce qui contribue au placement stable du récipient 16 sur l'élément distributeur 13.

Les picots 29 maintiennent en outre le composé chimique actif détergent solide ou pâteux 11 à distance de la membrane semi-perméable 23 durant l'utilisation du dispositif 10 pour ne pas empêcher la libération des substances parfumantes.

Le récipient auxiliaire 16 est placé sur l'élément distributeur 13 avec sa membrane semi-perméable 23 tournée vers la partie interne de l'élément distributeur 13. Dans cette position, la couche de protection 18 est donc atteinte par le flux d'eau de la chasse d'eau.

Dans le dispositif selon l'invention, le composé chimique actif libéré par l'élément distributeur et les substances volatiles parfumantes libérées par le récipient auxiliaire sont donc conservés séparément et n'interagissent pas entre eux, et sont libérés respectivement dans le flux de la chasse d'eau et dans l'environnement.

Le dispositif hygiénique selon l'invention a l'avantage de fournir un intense parfum dans l'air, et en particulier de parfumer et/ou désodoriser en continu indépendamment de l'action du flux de la chasse d'eau.

Ledit dispositif permet avantageusement l'emploi de substances volatiles, contenues dans un élément, pour obtenir un effet efficace parfumant et/ou désodorisant.

Enfin, l'activation de la libération des substances parfumantes par mise en contact du dispositif selon l'invention avec un flux d'eau est simple à effectuer, elle n'oblige pas à démonter le produit pour accéder au récipient auxiliaire et, enfin, elle ne produit pas de matériel d'emballage supplémentaire à éliminer ou recycler.

## Revendications

1. Dispositif hygiénique (10, 10') avec élément parfumant pour sanitaires, d'un type adapté pour être installé à l'intérieur et sur le bord des toilettes, comprenant, d'une part, un élément distributeur (13) d'au moins un composant chimique actif (11), qui relâche dans le flux de la chasse d'eau une quantité déterminée dudit composant actif, et, d'autre part, un récipient auxiliaire (16), le récipient auxiliaire comprenant un réservoir (26) contenant un élément contenant une ou des substances parfumantes volatiles **caractérisé en ce que** le réservoir comprend une membrane semi-perméable (23) qui permet la libération continue desdites substances volatiles dans l'air, ladite membrane (23) étant recouverte jusqu'à l'utilisation du dispositif hygiénique par une couche de protection (18) hydrosoluble, **en ce que** le récipient auxiliaire (16) est appliqué sur l'élément distributeur (13) par pression dans une base ou ouverture (22) de forme complémentaire à celle du récipient auxiliaire (16) et **en ce que** l'élément distributeur (13) comprend un récipient en matière plastique (13a) doté de multiples trous et/ou fissures (13b) pour le passage de l'eau et contenant ledit composé actif (11) qui est solide ou pâteux, ladite base ou ouverture (22) étant réalisée sur la partie antérieure dudit élément distributeur, le récipient auxiliaire (16) étant placé dans ladite base (22) avec la membrane semi-perméable (23) dirigée vers la partie interne de l'élément distributeur (13).

2. Dispositif selon la revendication 1 **caractérisé par le fait que** la membrane semi-perméable (23) comprend au moins une couche de polyéthylène perméable aux substances volatiles et est co-extrudée avec la couche de protection (18).

3. Dispositif selon la revendication 1 **caractérisé par le fait que** le récipient auxiliaire (16) est appliqué de façon fixe ou amovible sur l'élément distributeur (13).

4. Dispositif selon la revendication 1, **caractérisé par le fait que** la partie antérieure de l'élément distributeur (13) est pourvue d'au moins un picot (29) dépassant de la surface interne et adapté pour maintenir ledit composé actif solide ou pâteux (11) à distance du récipient auxiliaire (16).

5. Dispositif selon la revendication 4, **caractérisé par le fait que** le réservoir (26) dudit récipient auxiliaire (16) comprend un périmètre dont une partie (27) est munie d'au moins un trou (28) en position complémentaire du au moins un picot (29) pour un positionnement stable.

## Claims

1. Hygienic device (10, 10') having a perfuming element for sanitary appliances, of a type which is suitable for being fitted inside and on the edge of toilets, comprising on the one hand a distributor element (13) for at least one active chemical component (11), which distributor element (13) releases a given amount of said active component into the flow of the flushing water, and on the other hand an auxiliary chamber (16), the auxiliary chamber comprising a reservoir (26) containing an element comprising one or more volatile perfuming substances, **characterised in that** the reservoir comprises a semi-permeable membrane (23) which permits the continuous release of said volatile substances into the air, said membrane (23) being covered, until the hygienic device is used, by a water-soluble protective layer (18), **in that** the auxiliary chamber (16) is applied to the distributor element (13) by pressing into a base or opening (22) having a shape complementary to that of the auxiliary chamber (16) and **in that** the distributor element (13) comprises a chamber made of plastics material (13a) which is provided with a plurality of holes and/or slits (13b) for the passage of the water and which contains said active compound (11), which is solid or pasty, said base or opening (22) being formed on the front portion of said distributor element, the auxiliary chamber (16) being placed in said base (22) with the semi-permeable membrane (23) directed towards the inside of the distributor element (13).

2. Device according to claim 1, **characterised in that** the semi-permeable membrane (23) comprises at least one layer of polyethylene which is permeable to the volatile substances and is co-extruded with the protective layer (18).

3. Device according to claim 1, **characterised in that** the auxiliary chamber (16) is applied in a fixed or removable manner to the distributor element (13).

4. Device according to claim 1, **characterised in that** the front portion of the distributor element (13) is provided with at least one pin (29) which protrudes from the inside surface and is suitable for keeping said solid or pasty active compound (11) away from the auxiliary chamber (16).

5. Device according to claim 4, **characterised in that** the reservoir (26) of said auxiliary chamber (16) has a perimeter a portion (27) of which is provided with at least one hole (28) in a complementary position to the at least one pin (29) for stable positioning.

## Patentansprüche

1. Hygienevorrichtung (10, 10') mit einem parfümierenden Element für sanitäre Einrichtungen eines Typs, der angepasst ist, um im Inneren und am Rand von Toiletten montiert zu werden, umfassend einerseits ein Verteilerelement (13) wenigstens einer aktiven chemischen Verbindung (11), das eine bestimmte Menge der aktiven Verbindung in den Fluss der Wasserspülung freisetzt, und andererseits einen Hilfsbehälter (16), wobei der Hilfsbehälter ein Reservoir (26) umfasst, das ein Element enthält, das eine flüchtige parfümierende Substanz oder flüchtige parfümierende Substanzen enthält, **dadurch gekennzeichnet, dass** das Reservoir eine semi-permeable Membran (23) umfasst, die die kontinuierliche Freisetzung der flüchtigen Substanzen in die Luft erlaubt wobei die Membran (23) bis zur Verwendung der Hygienevorrichtung mit einer wasserlöslichen Schutzschicht (18) bedeckt ist, dass der Hilfsbehälter (16) an dem Verteilerelement (13) durch Drücken in eine Basis oder Öffnung (22) mit einer Form, die komplementär zu der des Hilfsbehälters (16) ist, angebracht ist, und dass das Verteilerelement (13) einen Behälter aus Kunststoffmaterial (13a) umfasst, der mit zahlreichen Löchern und/oder Spalten (13b) für den Durchgang des Wassers ausgestattet ist und der die aktive Verbindung (11), die fest oder teigig ist, enthält, wobei die Basis oder Öffnung (22) durch den vorderen Teil des Verteilerelements gebildet wird, der Hilfsbehälter (16) in der Basis (22) mit der semi-permeablen Membran (23) zu dem inneren Teil des Verteilerelements (13) ausgerichtet platziert ist.

2. Vorrichtung nach Anspruch 1, **dadurch gekennzeichnet, dass** die semi-permeable Membran (23) wenigstens eine Polyethylenschicht umfasst, die für flüchtige Substanzen permeabel ist und die mit der Schutzschicht (18) coextrudiert ist.

3. Vorrichtung nach Anspruch 1, **dadurch gekennzeichnet, dass** der Hilfsbehälter (16) in fester oder abnehmbarer Art an dem Verteilerelement (13) angebracht ist.

4. Vorrichtung nach Anspruch 1, **dadurch gekennzeichnet, dass** der vordere Teil des Verteilerelements (13) mit wenigstens einem Zahn (29) versehen ist, der aus der inneren Oberfläche herausragt und angepasst ist, um die feste oder teigige aktive Verbindung (11) vom Hilfsbehälter (16) beabstandet zu halten.

5. Vorrichtung nach Anspruch 4, **dadurch gekennzeichnet, dass** das Reservoir (26) des Hilfsbehälters (16) einen Umfang umfasst, von dem ein Teil (27) mit wenigstens einem Loch (28) in einer Position komplementär zu dem wenigstens einen Zahn (29) für eine stabile Positionierung ausgestattet ist.
